# EUROPEAN PATENT SPECIFICATION

(11) **EP 0 353 823 B1**
(45) Date of publication and mention of the grant of the patent: **12.01.1994**
(21) Application number: 89201985.2
(22) Date of filing: 28.07.1989
(51) Int. Cl.: A61K 39/015, G01N 33/569

(54) **Immunologically active peptide conjugate PPD-(NANP)n useful as an antimalaria vaccine**
Als Anti-Malaria-Impfstoff verwendbares immunologisch aktives Peptidkonjugat PPD-(NANP)n
Conjugué peptidique PPD-(NANP)n à activité immunologique utile comme vaccin antimalaria

(30) Priority: 05.08.1988 IT 2166688
(43) Date of publication of application: 07.02.1990
(73) Proprietor: ENIRICERCHE S.p.A., 20121 Milano (IT)
(72) Inventor: Verdini, Antonio Silvio, I-00015 Monterotondo Rome (IT); Pessi, Antonello, I-00199 Rome (IT); Del Giudice, Giuseppe, Hermance Geneva (CH); Lambert, Paul Henry, CH-1290 Versoix Geneva (CH)
(74) Representative: Fusina, Gerolamo

(56) References cited:
- EP-A- 0 209 643
- WO-A-85/00975
- WO-A-86/00911
- WO-A-86/05790
- US-A- 3 849 551
- CHEMICAL ABSTRACTS, vol. 108, no. 19, Columbus, OH (US); LACHMAN et al., p. 469, no. 165765#
- BIOCHEMICAL & BIOPHYSICAL RESEARCH COMMUNICATIONS, vol. 153, no. 1, 31 May 1988, Academic Press Inc., Duluth, MN (US); D. LISE et al.; pp. 31-38#

## Description

This invention relates to a synthetic immunogen useful in the preparation of an antimalaria vaccine and to a method for immunization against infections induced by malaria parasites which makes use of it.

More specifically, the invention relates to the conjugate of a synthetic peptide with the purified protein derivative of the immunologically active tuberculin which is useful in inducing protective immunity against the sporozoite stage of malaria.

The etiological agent of malaria is a protozoon of the Plasmodium genus which is transmitted to the host vertebrate by the sting of the anopheles mosquito.

Plasmodium falciparum (P.falciparum) is one of the most common, and causes most of the morbidity and mortality associated with the disease.

Malaria currently represents one of the most serious parasite diseases for man. In this respect it is calculated that every year it affects between 100 and 200 million individuals to result in a mortality which in initial infancy can attain 50% of cases.

There is therefore the requirement in this field for the development of an antimalaria vaccine able to give man protective immunity against this infection.

Malaria parasites develop, in accordance with a multi-stage cycle, by presenting the host with a very large number of antigen components, each form of development of the parasite containing different stage-specific antigens.

In an attempt to identify protective plasmodial antigens, the interest of researchers has been occupied by those exposed to the immune system and present both on the parasite surface and on the membrane of the infected red blood corpuscle.

The study of Plasmodium sporozoites has generated particular interest because a vaccine developed against this stage, if completely effective in man, would be able to prevent the development of the plasmodium in the host and therefore induce sterile immunity.

It is a known fact that administering sporozoites irradiated with X-rays to rodents, monkeys and man gives protective immunity which is stage-specific but not strain-specific [Cochrane A.H. et al (1980) Malaria Vol. 3 J.P.Kreier ed. Academic Press, New York pp. 163-202].

It is also known that incubating Plasmodium sporozoites with antisera obtained from animals immunized with X-ray irradiated sporozoites of the same species results in the formation of a precipitate on the surface of sporozoite (circumsporozoite precipitation reaction or CSP reaction).

The use of monoclonal antibodies has made it possible to identify specific antigens for said antisera.

These pertain to a family of polypeptides (circumsporozoite protein or CSP) which cover the entire surface of the sporozoite and induce a specific antibody response which confers protection against malaria infections.

Recent developments in the molecular biology field have made it possible to identify the antigen protein coding genes of plasmodium in its various stages of development and to determine the deduced amino acid sequences of said proteins.

In particular, the coding genes for the circumsporozoitic protein of P.falciparum (EP-A- 166410) have been cloned and sequenced.

An analysis of the characteristics of said proteins has shown the presence within said antigens of a central domain or immunodominant epitope consisting of units which repeat themselves a certain number of times.

For P.falciparum said immunodominant epitope consists of the tetrapeptide Asn-Ala-Asn-Pro (NANP) repeated 37 times and 4 quadruplets with the amino acid sequence Asn-Val-Asp-Pro (NVDP).

Various data indicate that synthetic vaccines based on the use of a synthesis peptide comprising or constituted of said sequence could be effective against malaria.

In this respect, said sequence is well preserved in all the P.falciparum isolates for which the CSP coding genes have been cloned and sequenced [V. Nussenzweig et al. Am. J. Trop. Med. Hyg. 35, 678 (1986)].

In addition, antisporocoite antibodies of P.falciparum react specifically with the synthetic peptide (NANP)₄₀ EP-A-209.643) and with the fusion protain R₃₂ₜₒₜ₃₂ obtained via recombinant DNA as described by M.F. Good et al. [J. Exp. Med. 164, 655 (1986)].

Again, D. Mazier et al. [Science 231, 156 (1986)] report that antipeptide antibodies inhibit in vitro the penetration of Plasmodium falciparum sporozoites into human liver cells.

However, studies carried out in various laboratories [G. Del Giudice et al. J. Immunol. 137, 2952 (1986); and the cited M.F. Good et al.] have shown that the antibody response towards the synthesis antigen (NANP)ₙ is genetically restricted in experimental animals, and only mice in which the gene I-A^{b} (tobi responders) is present in their genetic kit are able to produce specific antibodies.

In addition, the peptide (NANP)₄₀ and peptides comprising said sequence are poorly immunogenic if administered in the absence of conventional adjuvants.

The best antibody response has been obtained using complete Freund's adjuvant (CFA) which, however is unsuitable for administration to man.

It is known in the art that the immunogenicity of antigens which in themselves are poorly immunogenic can be improved by conjugating said antigens to a protein, known as the carrier protein, able to induce the production of antibodies directed specifically towards the antigen itself [H. A. Mitchison, Eur. J. Immunol. 1, 18 (1971)].

The function of the carrier, as has emerged from numerous studies, is to activate specific clones of T lymphocytes of helper type which, in their turn, supply suitable activation signals for the specific B cells of the antigen.

Those segments of the carrier recognised by the T lymphocytes constitute the T epitopes of the immunogen, while the antigen recognised by the B lymphocytes constitutes the B epitope.

Consequently an immunogen must simultaneously possess at least one B epitope and at least one T epitope for an immunized animal to be able to provide an antibody response to it.

In the case of P.falciparum the repetitive sequence (NANP)ₙ, which alone represents more than one third of the entire protein, constitutes the immunodominant B epitope of the sporozoitic development stage, and consequently must be conjugated to a carrier.

(NANP)ₙ peptide conjugates are known in the art which are useful in the development of an antimalaria vaccine and based essentially on the use of determined protein carriers, as described for example in the patent applications EP-A-192,626 and WO 86/05790.

Specifically, EP-A-192 626 describes and claims an Rₙₜₑₜ₃₂ conjugate obtained via recombinant DNA characterised in that the carrier (tet₃₂) consists of a segment of 32 amino acids coded by a fragment of the gene of the protein which gives resistance to tetracycline. WO 86/05790 describes and claims the conjugate of the synthetic peptide (NANP)₃ with the tetanus toxoid (TT).

Said conjugates are able to induce the formation of specific antibodies which recognise the CS protein of P.falciparum and inhibit in vitro the penetration of P.falciparum sporozoites into human liver cells.

However said known conjugates are not completely satisfactory for preparing an antimalaria vaccine, particularly in view of their limited immunogenic activity and the use of protein carriers which can induce the phenomenon known as epitope suppression.

In this respect, preliminary vaccination studies on healthy volunteers with the conjugates in question [D. H. Harrington et al., Nature, 328, 257 (1987); W.R. Ballon et al., Lancet, 1, 1277 (1987)] not only indicated a very limited protective capacity of these potential vaccines but also indicated that only individuals who had received repeatedly high doses of immunogen, using aluminium hydroxide as adjuvant, produced measurable levels of anti-(NANP)ₙ antibodies.

In addition, said antibodies had a half-life of only 27-28 days.

Again, carriers chosen from those generally used in this particular sector of the art can induce the formation of antibodies directed not only against the antigen B epitope but also against its own B and/or T epitopes.

In some cases the presence of antibodies towards the carrier epitopes can cause negative feed-back of the immune response, which appears as the suppression of anti-antigen antibody production.

This phenomenon, known as epitope suppression, has recently been observed and described by H;Etlinger et al., J.Immunol.,140, 626 (1988) in a subject which, having a high anti-TT antibody count, did not produce, after vaccination with the TT(NANP)₃ conjugate, any anti-(NANP)n antibodies.

An approach towards the preparation of a malaria vaccine is disclosed in US-A-3 849 551, in which Mycobacteria bovis, strain Calmette-Guérin bacillus (BCG), is employed as an adjuvant in conjunction with non-living vaccine materials: the BCG vaccine provides a potent stimulus for vaccination against malaria when used in conjuction with a plasmodial vaccine such as a Plasmodium knowlesi partially purified plasmodial vaccine fraction.

Another interesting approach can be seen in C.A. 108:165765q (1988) in which PPD (purified protein derivative of tuberculin) is proposed to raise antibodies by coupling PPD to peptides, with a view to raise antibodies to haptens and to raise T-cell responses to tumour cells.

The invention now proposes an immunologically active peptide conjugate capable of inducing, in the absence of adjuvant, in a previously BCG-sensitized mammal, an antibody-response which is cross-reactive towards the antiporozoite antigen of Plasmodium falciparum, characterized in that said peptide has the formula:

**PPD(NANP)**_{**n**}

wherein PPD is a purified protein derivative of tuberculin, N is Asparagine, A is Alanine, P is Proline and n is from 3 to 40.

An aspect of the invention is thus an antimalarial vaccine containing, as its active component, the peptide conjugate as defined hereinabove.

Consequentially, the invention makes it possible to immunize a mammal against malaria caused by Plasmodium falciparum, by administering to the mammal concerned, including humans negative to the tuberculin test, an immunologically active amount of Bacillus Calmette-Guérin at least two weeks prior to vaccination with the peptide conjugate defined hereinabove.

The invention will be better understood from the ensuing description and examples.

In particular, peptide conjugates according to the present invention can consist of a synthesis peptide having an amino acid sequence corresponding to that of the repeating immunodominant B epitope of the CSP of P.falciparum bound to the purified protein derivative (PPD) of tuberculin.

Preferably, said peptide has the amino acid sequence:

(NANP)ₙ

where N is asparagine, A is alanine. P is proline and n is between 3 and 40, and corresponding to the repeating sequence of the circumsporozoitic protein of P.falciparum.

In this respect, the development of a vaccine against said plasmodium, which causes most of the morbidity and mortality associated with malaria, is particularly desirable.

Said peptide is synthesized with high yield and high purity by the polycondensation process described and claimed in EP-A-209.643.

A peptide conjugate according to the present invention can be prepared by binding the synthetic peptide to the PPD protein carrier by operating in accordance with one of the known methods of the art.

According to a preferred embodiment of the present invention the synthetic peptide (NANP)₄₀ is bound to the tuberculin purified protein derivative, in the presence of gluteraldehyde as binding reagent, by the method described by Avrameas and Ternynck [Immunochemistry 6, 53, (1969)].

The PPD protein used as carrier can be chosen from those available commercially or can be prepared by boiling at 100°C the supernatant fraction of Mycobacterium tuberculosis followed by ultrafiltration and treatment with trichloroacetic acid.

A peptide conjugate prepared according to the present invention advantageously enables a high antibody response towards the CSP protein to be obtained in mice non-responding to the (NANP)₄₀ synthetic antigen who have been pretreated with bacillus Calmette-Guerin (BCG).

Moreover, when administered to mice without any adjuvant, said conjugate enables the same antibody counts to be obtained as when using the complete or incomplete Freund's adjuvant.

It has been found that the antibody response induced following immunization with a conjugate according to the present invention persists far 5-6 weeks and can be boosted by a third injection of the conjugate.

Again the character of susceptibility or resistance to Mycobacterium tuberculosis or BCG does not influence the antibody response towards said conjugate.

The use of PPD as carrier for B epitopes pertaining to the malaria parasite proteins is particularly advantageous, both because said protein is authorized for use on human beings and because in contrast to other known carriers, such as TT, it does not stimulate the production of anti-PPD antibodies.

This makes any appearance of the epitope suppression phenomenon extremely improbable.

Consequently, a peptide conjugate according to the present invention is particularly useful as an antimalaria vaccine.

The present invention makes it possible to obtain protective antimalaria immunization in mammals, including human subjects negative to the tuberculin test, by administering an immunologically effective dose of BCG at least two weeks prior to vaccination with a peptide conjugate according to the present invention. In this respect, from the results given in Example 4 it can be seen that said conjugates are unable to induce the formation of antisporozoite antibodies of Plasmodium if administered to mice not vaccinated with BCG or if administered less than two weeks after vaccination with BCG.

This is due to the fact that immunization with bacillus Calmette-Guerin stimulates T cells sensitive to PPD.

Consequently by administering a conjugate of said protein these T cells should be boosted, so inducing a more intense antibody response towards the B epitope conjugated to PPD.

In geographical areas where malaria is endemic there is considerable prevalence of infection by M.tuberculosis and therefore a positive result to the tuberculin test, it therefore being sufficient to vaccinate with a conjugate according to the present invention to obtain protective immunity against malaria.

The following examples illustrate but do not limit the invention.

### EXAMPLE 1

### Preparation of the polypeptide conjugate PPD-(NANP)₄₀

Conjugation between the polypeptide (NANP)₄₀ and the tuberculin purified protein derivative (PPD) was implemented using gluteraldehyde in accordance with the procedure described by Avrameas and Ternynck [Immunochemistry 6, 63 (1969)].

5 mg of commercial PPD (Staten Serum Institut, Copenhagen, Denmark) and 50 mg of (NANP)₄₀ polypeptide synthesized as described in the EP-A-209.643 were mixed in 5 ml of PBS (phosphate buffered saline), pH 7.5.

A 2% solution of gluteraldehyde in water was then added to a final concentration of 1%.

After incubation at ambient temperature (25°C) for 2 hours, the mixture was extensively dialysed against H₂O for 20 hours and lyophilized.

The conjugation reaction yield was 55% (determined by analysing the amino acid content). The resultant product was stored at -30°C as a solid and redissolved in PBS only at the time of use.

### EXAMPLE 2

### Vaccination of BALB/c mice with the PPD(NANP)₄₀ conjugate

Two groups of male BALB/c mice of 2-3 weeks who were non-responders to non-conjugated (NANP)₄₀ and susceptible to infection with bacillus Calmette-Guerin ) (BCG) were used.

0.5 ml of phosphate buffered saline solution of pH 7.8 (PBS 100 mM NaCl) containing 106 cFu of BCG (Pasteur Institute, Paris) were administered intraperitoneally (i.p.) to all the mice.

After 21 days the first group was immunized by i.p. injection with 0.5 ml of PBS containing 25 µg of PPD-(NANP)₄₀, while the second group was immunized by intramuscular injection at the base of the tail with the same quantity of conjugate in incomplete Freund's adjuvant (IFA).

The two groups then received a second and third administration of the conjugate 49 and 126 days after the first inoculation operating as stated heretofore.

The anti-(NANP)₄₀ antibody response was determined every one or two weeks by assaying animal serum samples diluted in PBS buffer by the ELISA using (NANP)₄₀ as specific binder fixed to the microtitration plate as described by Del Giudice et al. [J. Chem. Microbiol. 25, 91 (1987)].

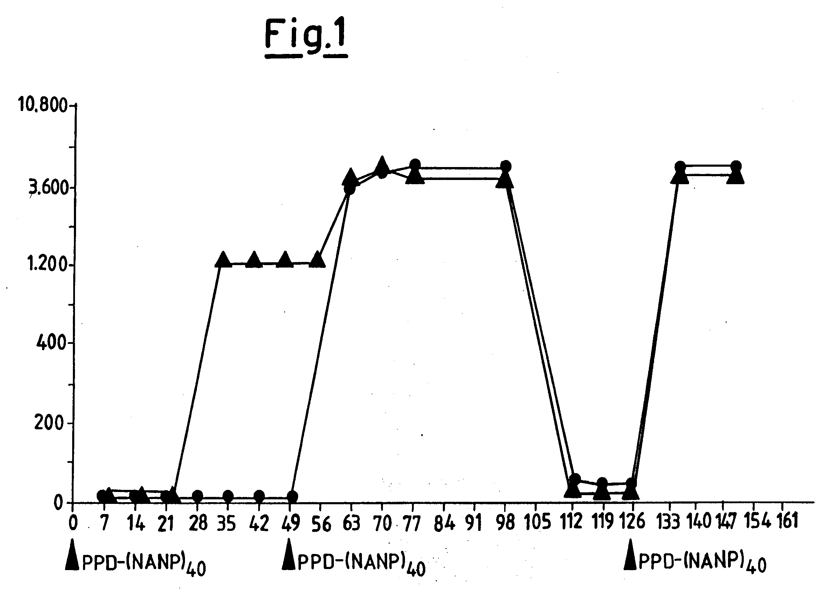

The results obtained are shown in Figure 1 where:
- ▲ indicates the values found for mice immunized with the PPD-(NANP)₄₀ conjugate in IFA;
- indicates the values found for mice immunized with the PPD-(NANP)₄₀ conjugate in PBS buffer alone;
- the vertical axis represents the reciprocal of serum dilutions;
- the horizontal axis represents time.

On the basis of the results shown in Figure 1 it can be observed that:
1) BALB/c mice immunized with BCG and PPD-(NANP)₄₀ in the presence of incomplete Freund's adjuvant produce anti-(NANP)₄₀ antibodies after the first conjugate injection.
   Mice vaccinated with BCG and PPD-(NANP)₄₀ in PBS alone produce anti-(NANP)₄₀ antibodies after the second conjugate injection.
   The antibody counts obtained in the two cases are identical and there are no antibodies pertaining to the IgM class.
2) The antibody response persists for 5-6 weeks and can be boosted by a third conjugate inoculation.

These results indicate that the PPD-(NANP)₄₀ conjugate is able to induce a high antibody response in non-responders animals immunized with BCG even in the absence of adjuvant.

### EXAMPLE 3

### Vaccination of BALB/c and C₃H/HeJ mice with the PPD-(NANP)₄₀ conjugate

Groups of BALB/c and C₃H/HeJ mice were used to investigate the influence of the susceptibility of BCG on vaccination with PPD-(NANP)₄₀ and the optimum time interval between immunization with BCG and immunization with the conjugate.

Said mice are all incapable of producing antibodies against the non-conjugated (NANP)₄₀ peptide in complete Freund's adjuvant (CFA), but whereas the BALB/c mice are susceptible to infection by BCG, the C₃H/HeJ mice are resistant.

The procedure of Example 2 was followed, immunizing all the mice firstly with BCG and then, at different time intervals from said inoculation, with the PPD-(NANP)₄₀ conjugate in PBS or in IFA.

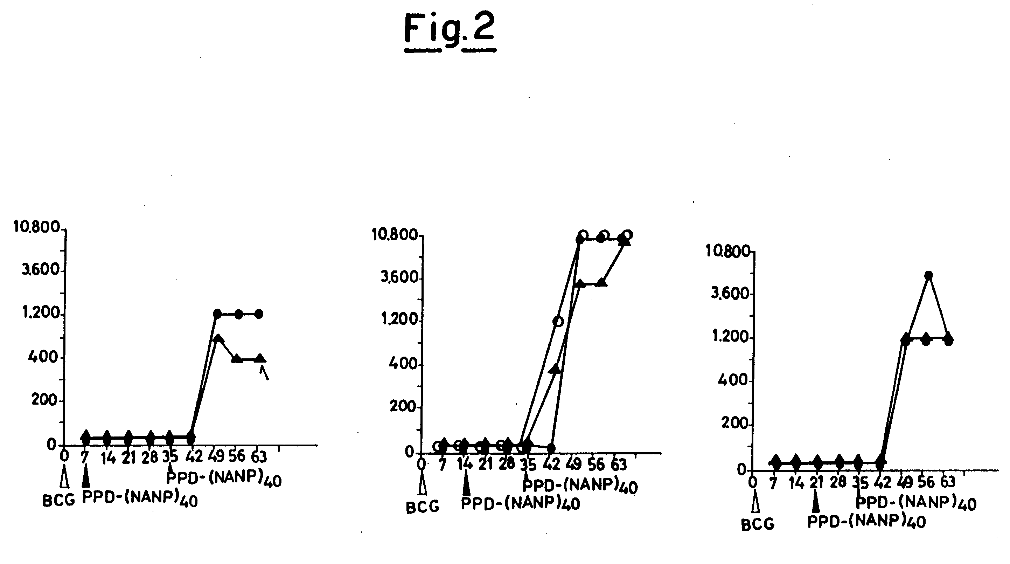

The results are shown in Figure 2 where:
● indicates the measurement of the anti-(NANP)₄₀ antibody levels in BALB/c mice immunized with PPD-(NANP)₄₀ in PBS;
▲ indicates the measurement of the anti-(NANP)₄₀ antibody levels in C₃H/HeJ mice immunized with PPD-(NANP)₄₀ in PBS; and
ϑ indicates the measurement of the anti-(NANP)ₙ antibody levels in BALB/c mice immunized with PPD-(NANP)₄₀ in IFA.

The vertical axis represents the reciprocal of the serum dilution and the horizontal axis represents the time intervals following BCG administration.

Figure 2 confirms the results obtained in Example 2 relative to the BALB/c mice and to the presence or absence of adjuvant.

It can also be seen that there is no specific antibody difference in the two strains of mice, and that the characteristic of susceptibility or resistance to BCG is therefore insignificant.

In addition, the highest antibody response was obtained by administering the PPD-(NANP)₄₀ conjugate in PBS 2-3 weeks after vaccination with BCG.

### EXAMPLE 4

Vaccination of C57BL/6 mice genetically responders to (NANP)₄₀ in CFA with the PPD-(NANP)₄₀ conjugate

The series of experiments described in Examples 2 and 3 was conducted on 5 groups of male C57BL/6 mice of age 2-3 weeks capable of developing an antibody response when immunized with (NANP)₄₀ in CFA.

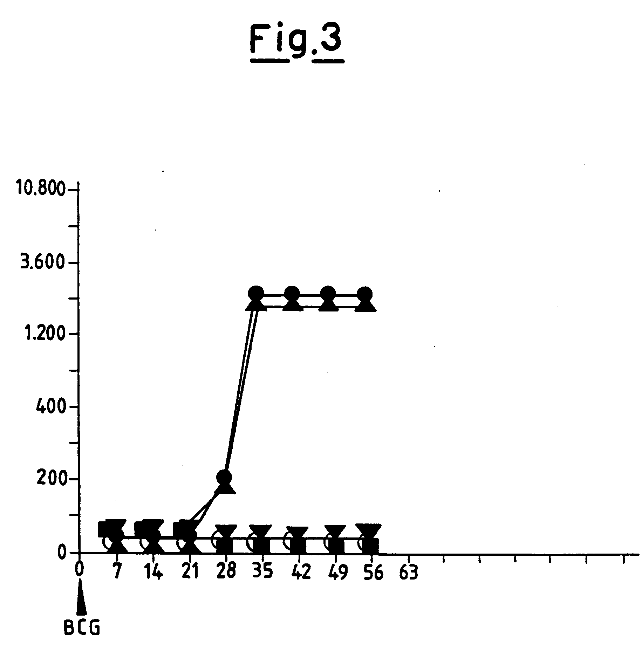

Figure 3 shows the anti-(NANP)₄₀ antibody response in different groups of C57BL/6 mice where:
ϑ indicates mice not immunized with BCG before administering the conjugate;
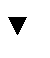 indicates mice immunized with PPD-(NANP)₄₀ but not previously immunized with BCG;
■ indicates mice immunized with the conjugate one week after administering BCG
▲ indicates mice immunized with the conjugate 2 weeks after administering BCG;
● indicates mice immunized with the conjugate 3 weeks after administering BCG.

The results shown in Figure 3 indicate that:
1) Mice vaccinated with PPD-(NANP)₄₀ in PBS produced anti-(NANP)₄₀ antibodies after the first injection if this was done 2-3 weeks after administering BCG.
2) No formation of anti-(NANP)₄₀ antibodies occurred if the mice were immunized with the conjugate one week after administering BCG (■) or if there was no such administration ( 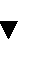 ).

## Claims (Claims for the following Contracting State(s): AT, BE, CH, DE, FR, GB, LI, LU, NL, SE)

1. An immunologically active peptide conjugate capable of inducing, in the absence of adjuvant, in a previously BCG-sensitized mammal, an antibody-response which is cross-reactive towards the antiporozoite antigen of Plasmodium falciparum, characterized in that said peptide has the formula:
**PPD-(NANP)**_{**n**}
wherein PPD is a purified protein derivative of tuberculin, N is Asparagine, A is Alanine, P is Proline and n is from 3 to 40.

2. An antimalarial vaccine containing, as its active component, the peptide conjugate as defined in Claim 1.

## Claims (Claims for the following Contracting State(s): ES, GR)

1. Process for preparing an immunologically active peptide conjugate capable of inducing, in the absence of adjuvant, in a previously BCG-sensitized mammal, an antibody-response which is cross-reactive towards the antiporozoite antigen of a Plasmodium falciparum, characterized in that it is prepared by conjugating a synthetic peptide consisting of the sequence:
**H-(NANP)**_{**n**} **-OH**
wherein N is Asparagine, A is Alanine, P is Proline and n is from 3 to 40, with a purified protein derivative (PPD) of tuberculin.

## Patentansprüche (Patentansprüche für folgende(n) Vertragsstaat(en): AT, BE, CH, DE, FR, GB, LI, LU, NL, SE)

1. Immnologisch aktives Peptid-Konjugat, welches befähigt ist, in einem zuvor BCG-sensibilisierten Säugetier in Abwesenheit eines Adjuvans eine Antikörper-Antwort zu induzieren, welche gegenüber dem Anti-Sporozoiten-Antigen von Plasmodium falciparum kreuzreaktiv ist, dadurch gekennzeichnet, daß dieses Peptid die Formel:
PPD-(NANP)ₙ
hat, worin PPD ein gereinigtes Proteinderivat des Tuberkulins ist, N für Asparagin steht, A für Alanin steht, P für Prolin steht und n eine Zahl von 3 bis 40 ist.

2. Anti-Malaria-Vakzine, welche als Wirkstoffkomponente das im Anspruch 1 definierte Peptid-Konjugat enthält.

## Patentansprüche (Patentansprüche für folgende(n) Vertragsstaat(en): ES, GR)

1. Verfahren zur Herstellung eines immunologisch aktiven Peptid-Konjugates, welches befähigt ist, in einem zuvor BCG-sensibilisierten Säugetier in Abwesenheit eines Adjuvans eine Antikörper-Antwort zu induzieren, welche gegenüber dem Anti-Sporozoiten-Antigen von Plasmodium falciparum kreuzreaktiv ist, dadurch gekennzeichnet, daß dieses Peptid-Konjugat durch Konjugieren eines synthetischen Peptides, welches aus der Sequenz
H-(NANP)ₙ-OH
besteht, worin N für Asparagin steht, A für Alanin steht, P für Prolin steht und n eine Zahl von 3 bis 40 ist, mit einem gereinigten Proteinderivat (PPD) des Tuberkulins hergestellt wird.

## Revendications (Revendications pour l'(les) Etat(s) contractant(s) suivant(s): AT, BE, CH, DE, FR, GB, LI, LU, NL, SE)

1. Conjugué peptidique immunologiquement actif, capable d'induire, en l'absence d'un adjuvant, sur un mammifère préalablement traité avec le BCG, une réponse-anticorps qui est interactive vis-à-vis de l'antigène antisporozoïte du Plasmodium falciparum, caractérisé par le fait que ledit peptide répond à la formule :
PPD - (NANP)ₙ
dans laquelle PPD est une protéine purifiée dérivée de la tuberculine, N est l'asparagine, A est l'alanine, P est la proline et n vaut 3 à 40.

2. Vaccin anti-malaria contenant comme principe actif, le conjugué peptidique tel que défini dans la revendication 1.

## Revendications (Revendications pour l'(les) Etat(s) contractant(s) suivant(s): ES, GR)

1. Procédé pour préparer un conjugué peptidique immunologiquement actif, capable d'induire, en l'absence d'adjuvant, sur un mammifère préalablement traité avec le BCG, une réponse-anticorps qui est interactive vis-à-vis de l'antigène antisporozoïte du Plasmodium falciparum, caractérisé par le fait qu'il est préparé en conjuguant un peptide synthétique constitué de la séquence :
H - (NANP)ₙ - OH
où N est l'asparagine, A l'alanine, P la proline et n vaut 3 à 40, avec une protéine purifiée (PPD) dérivée de la tuberculine.
